# EUROPEAN PATENT APPLICATION

(11) **EP 1 354 892 A1**
(43) Date of publication of application: **22.10.2003**
(21) Application number: 02076552.5
(22) Date of filing: 19.04.2002
(51) Int. Cl.: C07K 14/705, G01N 33/68, A61K 38/17, A61P 25/28

(54) **Peptides capable of inducing attraction of the axonal growth and their use for treating neurodegenerative diseases**

(71) Applicant: CENTRE NATIONAL DE LA RECHERCHE SCIENTIFIQUE (CNRS), 75016 Paris (FR); Université de la Méditerranée - Aix-Marseille II, 13007 Marseille (FR)
(72) Inventor: Rougon, Geneviève, 13009 Marseille (FR); Castellani, Valérie, 13009 Marseille (FR)
(74) Representative: Warcoin, Jacques

(57) **Abstract**

The present invention is directed to peptides comprising the sequence ASNKL (SEQ ID N° 1), inducing attraction of the axonal growth in presence of the semaphorin (Sema3A), L1 and NP-1 proteins and their use for the manufacture of a drug for neuronal/axonal regeneration or for treating neurodegenerative diseases. The present invention also includes a method for identifying a compound able to bind the NP-1, a method for identifying a compound able to revert the repulsory effect of Sema3A on the axonal growth from a suited cell, or methods to screen for molecules capable of blocking Sema3A- induced endocytosis.

## Description

The present invention is directed to peptides comprising the sequence ASNKL (SEQ ID N° 1), inducing attraction of the axonal growth in presence of the semaphorin (Sema3A), L1 and NP-1 proteins and their use for the manufacture of a drug for neuronal/axonal regeneration or for treating neurodegenerative diseases. The present invention also includes a method for identifying a compound able to bind the NP-1, a method for identifying a compound able to revert the repulsory effect of Sema3A on the axonal growth from a suited cell, or methods to screen for molecules capable of blocking Sema3A- induced endocytosis.

Axon pathfinding is regulated by a number of chemotropic and cell-contact guidance cues that act as attractants or repellents for developing neuronal projections. At choice points along the pathways, a complex series of events may take place to ensure appropriate decisions made by the growth cone (Cook et al., Curr Opin Neurobiol., 8:64-72, 1998). In particular, this has been documented for commissural projections where a highly dynamic mechanism of up- and down-regulation of axonal responses to guidance cues occurs to sequentially orientate the axons towards, across, or away from the midline. These events were found to involve cell adhesion molecules of the immunoglobulin (Ig) superfamily as well as secreted factors of the netrin, slit and semaphorin families (Stoeckli and Landmesser, Opin Neurobiol, 8:73-79, 1998). In rodents, loss of function of the Ig superfamily cell adhesion molecule (IgCAM) L1 has implicated this protein in axonal guidance at the pyramidal decussation (Cohen et al., Curr Biol, 8:26-33, 1998). This particular stage in corticospinal pathfinding has been characterized by two major events: axon growth across the midline followed by a switch from a ventral to a dorsal trajectory in the spinal cord. L1 is likely to be important for each of these steps as it may be required as a cell-contact cue for crossing the midline (Cohen et al., 1998). In addition, L1 has also been shown *in vitro* to be required for axons to respond to a ventrally-secreted semaphorin, Sema3A, that triggers the dorsalization of the pathway (Castellani et al., Curr Biol., 8:26-33, 2000).

Recent studies suggest that semaphorins signal through multi-molecular receptor complexes, containing neuropilin-1 (NP-1) and /or neuropilin-2 (NP-2) as specific ligand binding subunits (Raper, Curr Opin Neurobiol., 10:88-94, 2000), and plexins as signal transducers (Tamagnone and Comoglio, Trends Cell Biol., 10:377-383, 2000). L1 has also been shown to associate with NP-1 and is required as part of the Sema3A receptor complex for axon guidance responses (Castellani et al., 2000). A functional link between L1 and NP-1/Sema3A signaling is supported by the finding that a soluble L1Fc chimera switches Sema3A-induced chemorepulsion to attraction (Castellani et al., 2000). Previously it has been suggested that growth cones can switch between attraction and repulsion via a modulation of internal cyclic nucleotide levels (Song et al., Science, 281:1515-1518, 1998). How binding of soluble L1 on the cell surface can induce such a switch is largely unclear. L1 displays a highly complex pattern of homophilic and heterophilic interactions. In addition to L1 and NP-1, as components of the Sema3A receptor complex, axons express a number of other binding partners that could potentially be the receptor for L1Fc in the Sema3A reversion. These include other Ig CAMs such as F3/contactin, and TAG-1/axonin-1, α3-β5 integrins as well as several ECM proteins, such as laminin and some chondroitin sulfate proteoglycans (Brümmendorf and Rathjen, Curr Opin Neurobiol, 6:584-593, 1996). Furthermore, L1 expression on the growth cone is highly dynamically regulated. Following ligand activation, L1 is internalized, transported into vesicles to be recycled on the cell surface (Kamiguchi and Lemmon, J. Neurosci., 20:3676-3686, 2000).

A clear requirement for L1 in axon development is reflected by the variety of neurological abnormalities associated with L1 mutations in the human disorder referred to as X-linked hydrocephalus, MASA syndrome or SPG type 1 (hydrocephalus, enlarged ventricles, corpus callosum agenesis, corticospinal tract hypoplasia, Rosenthal et al., Nat Genet. 2:107-112, 1992 ; Vits et al., Nat Genet., 7:408-413, 1994 ; Jouet et al., Nat Genet., 7:402-407, 1994). Mutations in L1 that give rise to these disorders include many that would truncate the mature protein and eliminate cell surface expression. More subtle missense mutations within L1 have been shown to have multiple effects on L1 function disrupting intracellular protein trafficking or ligand binding or both (De Angelis et al., EMBO J., 18:4744-4753, 1999). The possibility that these mutations affect axonal responses to Sema3A has hitherto not been examined.

Surprisingly, the inventors, after having dissected the molecular mechanisms underlying the switch of axonal responses to Sema3A by soluble L1, using L1Fcs that contain pathological missense mutations, have demonstrated that binding of L1Fc (also noted L1-Fc in the present description) to NP-1 but not to L1 or to two other Ig CAMs expressed on growth cones is crucial for L1Fc to activate the conversion process. They have also demonstrated that binding and co-immunoprecipitation studies implicate the region containing mutation L120V in Ig domain 1 of L1 in both *trans* and *cis* L1/NP-1 complex formation. Moreover, they have demonstrated that a peptide composed of the (F)ASNKL₁₂₀ amino-acid sequence was sufficient to reverse Sema3A-mediated repulsion.

So, in a first aspect, the present invention is directed to a peptide comprising the sequence ASNKL (SEQ ID N° 1), preferably, inducing attraction of the axonal growth, in particular in presence of the semaphorin (Sema3A), L1 and NP-1 proteins.

In a preferred embodiment of this first aspect, the present invention includes the peptides comprising the sequence SEQ ID N° 1 having no more than 10 amino acids residues.

The peptide consisting of SEQ ID N° 1 and a peptide comprising or consisting of the sequence of FASNKL (SEQ ID N° 2) or FASNKLGTAM (SEQ ID N° 3) are particularly preferred.

An inducer of axonal growth comprising one of the peptide of the present invention forms also part of the first aspect of the present invention.

In a second aspect, the present invention is directed to a method for identifying a compound able to bind the NP-1 protein, comprising the steps of:
a) contacting said compound with a sample comprising the NP-1 protein, before, after or concomitantly with contacting said sample with the L1-Fc protein or with a peptide according to the present invention, particularly with a peptide comprising or consisting of the sequence SEQ ID N° 1 , SEQ ID N° 2 or SEQ ID N° 3,
b) studying the binding between NP-1 and L1Fc or said peptide according to the invention, the binding of said compound to the NP-1 protein being deduced from the alteration of the binding of NP-1 with L1-Fc or said peptide according to the invention observed in absence of said compound.

In a preferred embodiment of this second aspect, the present invention is related to the method for identifying a compound able to bind the NP-1 protein according to the present invention, wherein said sample also comprises the L1 protein.

In a further preferred embodiment of this second aspect, the present invention is related to the method for identifying a compound able to bind the NP-1 protein according to the present invention, wherein said NP-1 protein is a recombinant protein.

In a further preferred embodiment of this second aspect, the present invention is related to the method for identifying a compound able to bind the NP-1 protein according to the present invention, wherein said sample comprises a cell expressing the NP-1 protein at its surface.

In a further preferred embodiment of this second aspect, the present invention is related to the method for identifying a compound able to bind the NP-1 protein according to the present invention, wherein said sample comprises a cell expressing the NP-1 and L1 proteins at its surface.

In a further preferred embodiment of this second aspect, the present invention is related to the method for identifying a compound able to bind the NP-1 protein according to the present invention, wherein said L1-Fc protein or said peptide according to the invention is labeled and said binding of NP-1 protein with said L1-Fc protein or said peptide according to the invention is assayed by level of signal associated with said NP-1 protein.

The inventors have shown that L1/NP-1 *cis* interaction is necessary to the endocytosis of the Sema3A receptor complex associated with a repulsive response whereas L1/NP-1 *trans* interaction prevents it. This later interaction activates nitric oxide (NO) synthase and cGMP guanylyl cyclase. As indicated in the following examples, it has been identified molecular mechanisms involved in switching Sema3A repulsion to attraction and points to endocytosis as critical in determining the nature of axonal responses to guidance cues. Also it is provided a new explanation for the effects of human pathological mutations in the X-linked L1 gene.

So, in a particularly preferred embodiment of this second aspect, the present invention is related to the method for identifying a compound able to bind the NP-1 protein according to the present invention, wherein said alteration of binding of NP-1 with L1-Fc or said peptide according to the invention is assayed by measuring a secondary signal appearing in the presence of said binding.

In this particularly preferred embodiment of this second aspect, it is preferred that said secondary signal is induced in the presence of Sema3A.

In this particularly preferred embodiment of this second aspect, it is further preferred that said secondary signal is the reversion of the repulsory effect of Sema3A on axonal growth.

In this particularly preferred embodiment of this second aspect, it is also preferred that said secondary signal is the activation of NO synthesis.

In this particularly preferred embodiment of this second aspect, it is also preferred that said secondary signal is an increased production of cGMP.

In a third aspect, the present invention is directed to a method for attracting/modulating the direction of axonal growth by a cell suited to promote axonal growth, submitted to a Sema3A flow, comprising the step of contacting said cell with the peptide or the inducer according to the present invention.

In a fourth aspect, the present invention is directed to a method for identifying a compound able to revert the repulsory effect of Sema3A on the axonal growth from a suited cell, comprising the steps of contacting said cell expressing L1 and NP-1 at its surface with said compound in the presence of Sema3A, and studying the increase in NO synthesis in said cell.

In a fifth aspect, the present invention is directed to a method to screen for molecules that prevent the internalization of the dextran induced by the Sema3A treatment or to screen for molecules capable of blocking Sema3A- induced endocytosis by cells expressing L1 and NP-1 proteins at their surface characterized in that said method comprises the following steps of:
a) culturing cells expressing L1 and NP-1 in the presence of Sema3A, a labelled dextran and the molecule to be assayed ; and
b) visualizing or determining if said labelled dextran has been internalized into the cells ;
c) selecting the assayed molecule if no uptake of the labelled dextran by the cells could be detected.

In a preferred embodiment, said cells in step a) are recombinant cell lines transfected with expression vectors capable of expressing the L1 and NP-1 proteins at their surface or neuronal cells which naturally co-express the L1 and NP-1 proteins.

Such vectors will be prepared according to the methods commonly used by persons skilled in the art, and the clones resulting therefrom may be introduced into an appropriate host by standard methods such as, for example, lipofection, electroporation or heat shock.

In a further preferred embodiment, said labelled dextran in step a) is selected from the group consisting of rhodamine-dextran, FITC-dextran or dextran coupled to fluorescent molecules.

In a sixth aspect, the present invention is directed to a method to screen for molecules capable of blocking Sema3A- induced co-internalization of L1 and NP-1 proteins, to screen molecules capable of maintaining cell surface expression of L1 and NP-1 proteins or to screen molecules capable of blocking Sema3A- induced endocytosis characterized in that said method comprises the following steps of:
a) culturing cells expressing L1 and NP-1 in the presence of Sema3A and the molecule to be assayed ; and
b) visualizing or determining if said L1 and NP-1 proteins have been cointernalized into the cells using an immunocytochemical method in presence of antibodies, mono- or polyclonal, if appropriate, labelled, directed against L1 and NP-1 proteins ;
c) selecting the assayed molecule if L1 and NP-1 proteins could be detected at the surface of the cells.

In a preferred embodiment, said cells in step a) of the method of that sixth aspect according to the present invention, are recombinant cell lines transfected with expression vectors capable of expressing the L1 and NP-1 proteins at their surface or neuronal cells which naturally co-express the L1 and NP-1 proteins.

By immunocytochemical methods, it is intended to mean any immunocytochemical methods known by the skilled person which are capable of specifically detecting the presence of L1 and NP-1 at the surface of a cell using antibodies directed specifically against these L1 and NP-1 proteins.

Specific polyclonal antibodies may be obtained from a serum of an animal immunized against these L1 and NP-1 proteins. The specific monoclonal antibodies may be obtained according to the conventional hybridoma culture method described by Köhler and Milstein, 1975.

Said anti L1 and/or NP-1 antibodies, or Fab or F(ab')2 fragments thereof, may also be in the form of immunoconjugates or of labelled antibodies so as to obtain a detectable and/or quantifiable signal and so constitute a means for the immunocytochemical analysis of the expression of the L1 and NP-1 proteins at the surface of cells, for example by immunofluorescence, gold labelling, enzymatic immunoconjugates.

In a seventh aspect, the present invention is directed to use of the peptides or the inducer according to the present invention, or the molecules capable of blocking Sema3A- induced endocytosis selected by the method according to the present invention, for the manufacture of a drug for neuronal/axonal regeneration or for treating neurodegenerative diseases.

Finally, the present invention is directed to use of the peptides or the inducer according to the present invention, as a targeting agent, in the manufacture of a composition to be used in cell therapy.

The peptides or inducers according to the present invention as active ingredients of a medicament will be preferably in soluble form, combined with a pharmaceutically acceptable vehicle.

Such peptides, inducers or selected molecules capable of blocking Sema3A-induced endocytosis according to the present invention and which can be used as a medicament offer a new approach of treatment requiring or linked to neuronal/axonal regeneration, such as medicament for preventing and/or treating neurodegenerative diseases.

Preferably, these peptides, inducers or selected molecules capable of blocking Sema3A- induced endocytosis according to the present invention will be administered by the systemic route, in particular by the intravenous route, by the intramuscular or intradermal route or by the oral route.

Their modes of administration, optimum dosages and galenic forms can be determined according to the criteria generally taken into account in establishing a treatment suited to a patient, such as for example the age or body weight of the patient, the seriousness of his general condition, the tolerance to treatment and the side effects observed, and the like.

Other characteristics and advantages of the invention appear in the remainder of the description with the examples and figures whose legends are represented below.

### Figures legends

### Figures 1A to 1F: Co-cultures of cortical slices and ventral spinal cord explants in the presence of mutated L1Fc chimeras

Figure 1A: L1Fc chimeras with individual missense mutations affecting distinct Ig domains selected for the study.

Figure 1B: Scheme of the co-culture system of cortical slice and ventral spinal cord explant.

Figure 1C: The microphotographs illustrate the preferential growth of axons towards the source of Sema3A, in the presence of several mutant L1 chimeras. In contrast, the chimera carrying the L120V mutation was unable to switch the repulsive activity of Sema3A to attraction. In this case growth is directed away from the Sema3A source as in the control. Scale bar: 50 µm.

Figure 1D: H210Q, E309K, D598N, A426D and G121S mutant L1Fc chimeras were able to reverse Sema3A repulsion to attraction in the co-culture assay as indicated by the positive guidance indices.

Figures 1E and 1F: The histograms represent the number of axons growing towards (open bars) and away (filled bars) from the spinal cord explants (figure 1F) and their length (figure 1E). * Statistical difference in axon growth between the side facing the spinal cord explant (towards) and the side opposed to it (away) with P<0.0001.

### Figures 2A and 2B: Homophilic binding of mutant L1 chimeras to wild-type L1

Figure 2A: Binding of mutated L1Fc protein to wild-type L1 expressed in COS cells and detected by immunofluorescence analysis. Cell surface L1 was detected using Texas red-conjugated secondary antibodies and mutant L1Fc chimeras using FITC-conjugated secondary antibodies. A426D, H210Q and L120V mutated chimeras bound to wild-type L1 whereas G121S did not. Scale bar: 5 µm.

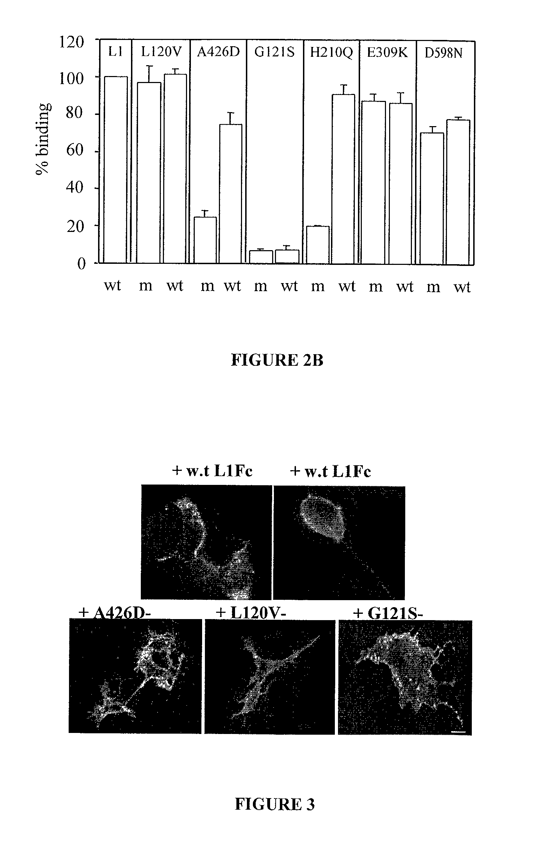

Figure 2B: Homophilic binding in wild-type/mutant and mutant/mutant combinations is compared by FACS analysis in the two-color aggregation assay. The histogram represents the percentage of mixed aggregates formed at the 30-minute time-point with values expressed as percent wild-type binding and error bars represent the SEM of 3 independent experiments. Wild-type L1-Fc is 100 %. For mutations A426D and H210Q, increased mutant to wild-type compared with mutant to mutant binding was observed, whereas binding of G121S remained extremely low for both combinations. wt: wild-type, m: mutant.

### Figure 3: Binding assay with L1Fcs on Plexin-A1 and NP-1 expressing cells

Cell surface expression of L1, Plexin-A1 and NP-1 was detected using Texas red-conjugated secondary antibodies. Mutant L1Fc chimeras were visualized using FITC-conjugated secondary antibodies. Wild-type L1Fc bound to L1 but not Plexin-A1-expressing cells. A426D and G121S L1Fc chimeras bound to NP-1 expressing cells, whereas the L120V chimera did not. Scale bar: 5µm.

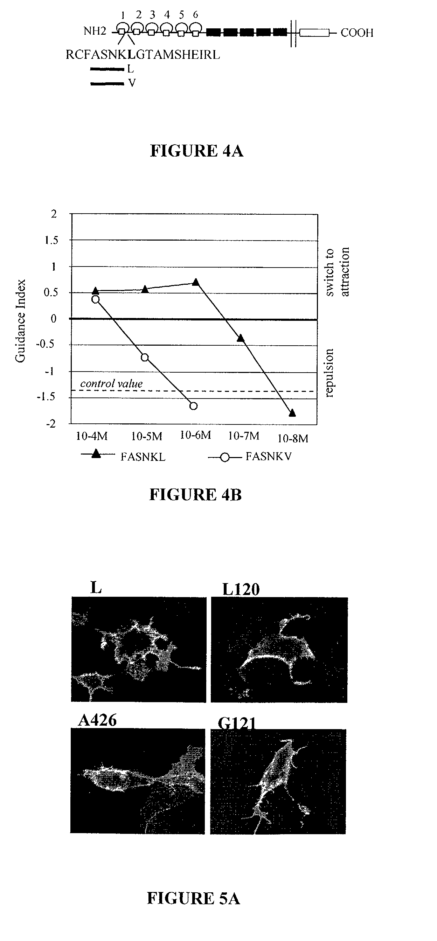

### Figures 4A and 4B: Switching Sema3A repulsion by a mimetic peptide of L1

Figure 4A: Part of the amino-acid sequence of L1 Ig1 domain. Six-amino-acid peptides containing either the native leucine or the mutant valine 120 residue were synthesized.

Figure 4B: Guidance induces in the co-culture assay for 5 different concentrations of the peptides. Note that at 10⁻⁵ and 10⁻⁶M, the peptide with C-terminal leucine switched Sema3A repulsion to attraction whereas the C-terminal valine did not.

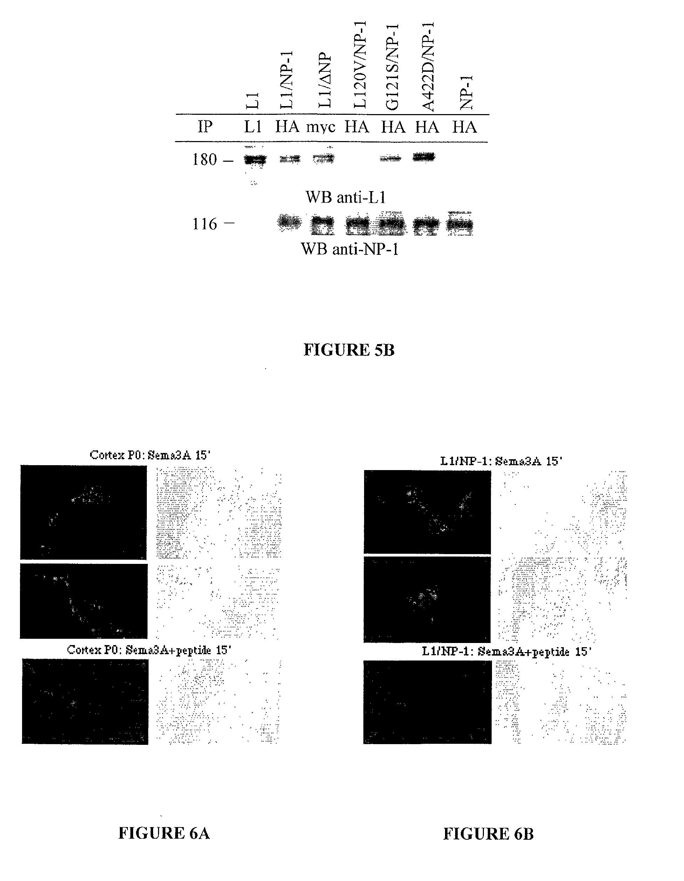

### Figures 5A and 5B: Effects of pathological L1 mutations on the formation of L1-NP-1 receptor complex

Figure 5A: Expression plasmids encoding full-length L1 containing missense mutations were transfected into COS7 cells and cell surface expression was detected by immunofluorescence analysis. As illustrated, mutant L1 constructs were expressed on the cell surface. Scale bar: 5 µm.

Figure 5B: Co-immunoprecipitation experiments. Cells transfected with NP-1 lacking its cytoplasmic domain (ΔNP-1) and full-length L1 were lysed and proteins were precipitated with anti-myc antibodies (see methods). Immunoblots were probed with anti-L1 and anti-NP-1 antibodies. Both L1 and NP-1 were detected in the precipitates, indicating that the *cis*-interaction involves the extracellular domains of each protein. Western blot analysis of cell lysate immunoprecipitated with anti-HA antibodies is shown for double transfectants expressing mutant L1 constructs (L120V, G121S or A426D) and full-length NP-1. L1 proteins with either the G121S or the A426D mutation co-immunoprecipitated with NP-1 whereas L120V -L1 did not.

### Figures 6A and 6B: Peptide mimetic of soluble L1 blocks Sema3A-induced endocytosis

Figure 6A: Neonatal cortical cells were incubated with Sema3A ("Cortex P0:Sema3A 15"'). The treatment induced internalization of Rhodamine-Dextran that accumulated along axons and around the soma. Internalization was blocked when Sema3A was applied in combination with the L1 mimetic peptide ("Cortex P0:Sema3A + peptide 15"'). Uptake of Rhodamine-Dextran was not detected in control conditions and in cultures treated with the peptide alone (not shown). Scale bar: 10 µm.

Figure 6B: COS7 cell co-expressing L1 and NP-1 also internalized Rhodamine-Dextran when treated with Sema3A ("L1/NP-1: Sema3A 15"'). This process was blocked by a Sema3A/peptide treatment ("L1/NP-1: Sema3A + peptide 15"'). As observed in cortical cell cultures, no endocytosis could be detected in control conditions or in cells treated with the peptide alone (not shown).

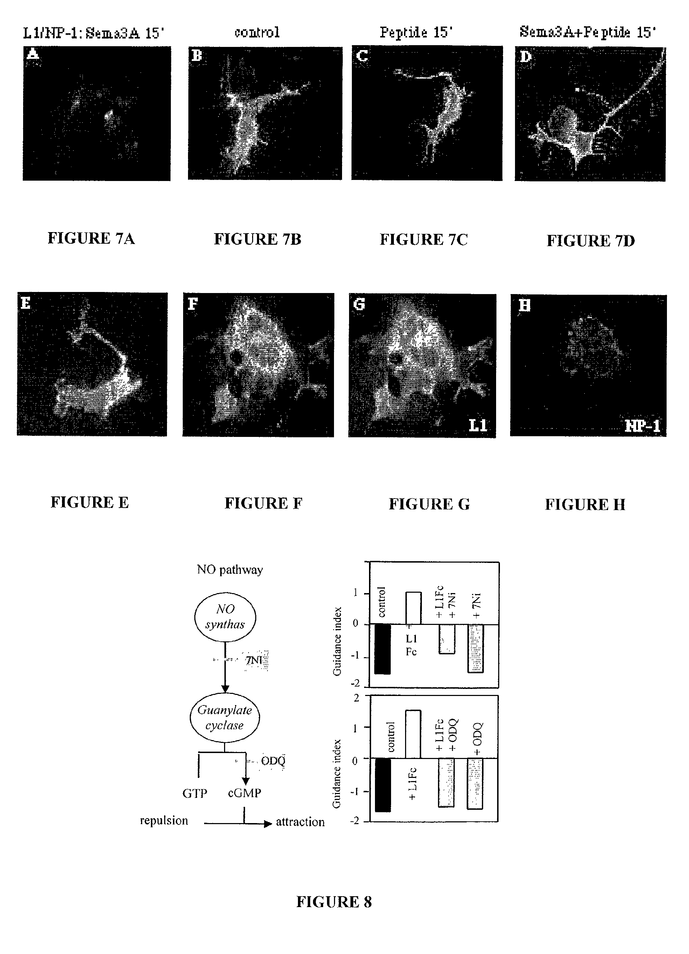

### Figures 7A to 7H: L1 ensures endocytosis of the Sema3A receptor complex

Figure 7A: Cell surface immunolabeling of L1 and NP-1 was undetectable after Sema3A treatment.

Figures 7B and 7C: In contrast, L1/NP-1 cell surface expression was observed in control condition (figure 7B) and after treatment with the peptide alone (figure 7C).

Figures 7D and 7E: When cells were treated with Sema3A in combination with the peptide, L1 and NP-1 were detected on the cell membrane, as illustrated in with these two panels.

Figures 7F to 7H: After cell permeabilization, co-localized spots of L1 and NP-1 immunolabeling were observed, indicating that loss of cell surface expression in figure 7A was due to internalization of the two proteins.

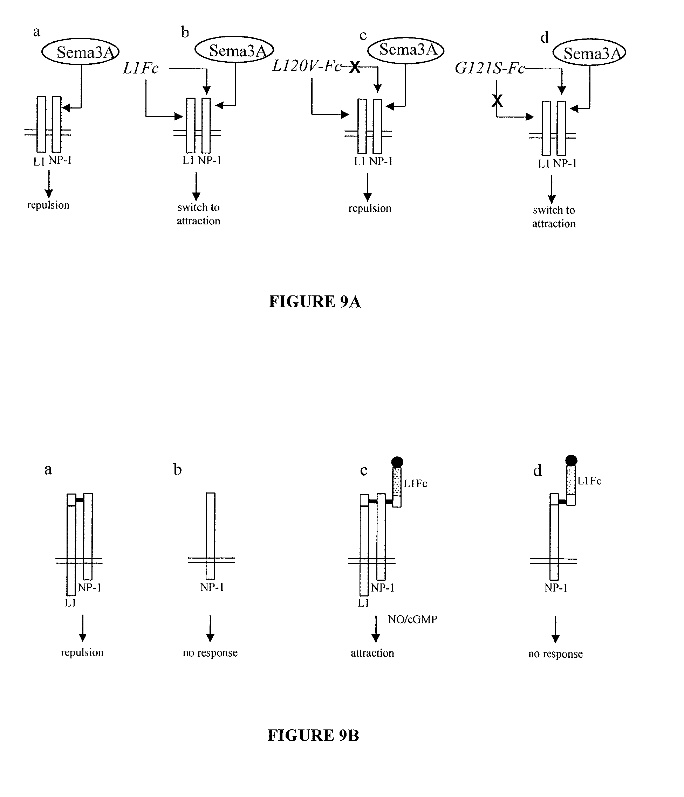

### Figure 8: Activation of the NO/cGMP pathway in the reversion of Sema3A repulsion

Pharmacological blockade of soluble guanylyl cyclase (with ODQ) and nNOS (with 7NI) in the co-culture of cortical slices and ventral spinal cord explants. In the presence of ODQ and 7NI alone, axons were repelled by the Sema3A source however, in the presence of L1Fc alone, axons switched from repulsion (negative guidance index) to attraction for the Sema3A source (positive guidance index). After addition of ODQ and 7NI in combination with L1Fc, axons no longer switched from repulsive to attractive responses. The number and length of cortical axons growing away from and towards the spinal cord explant were quantified for each condition. The number and length of axons were significantly reduced on the side facing the spinal cord explants, compared to the opposite side in ODQ, 7NI, ODQ+L1Fc, 7NI+L1Fc and control conditions. In the presence of L1Fc alone, axons were attracted, as indicated by the higher number and length of axons growing towards the spinal cord explant. Thus, ODQ and 7NI prevented the L1Fc induced switching of Sema3A from repulsion to attraction. Statistical analysis was conducted using the ANOVA test. * p<0.0001. Bars in the histograms: growth directed away (black bars) and towards (white bars) the spinal cord explant.

### Figures 9A and 9B: Models for L1-NP-1 interaction and modulation of the Sema3A signal

Figure 9A: The diagrams represent L1-L1 and L1/NP-1 *trans* interactions and the effects of missense mutations on the modulation of Sema3A signaling. (a) *cis* interaction of L1 with NP-1 triggers the repulsive Sema3A signal. (b) Soluble L1 (L1Fc) binds both L1 and NP-1 on the growth cone and switches Sema3A repulsion to attraction. (c) Soluble L1 containing the L120V mutation binds L1 but no longer NP-1 and fails to reverse Sema3A repulsion. (d) G121S, a mutation that destroys homophilic binding, does not affect L1 binding to NP-1 or alter the ability of the chimera to induce the switch. This shows that interaction of soluble L1 with NP-1 but not with L1 is crucial for the reversion process.

Figure 9B: A model for the interaction of L1 with NP-1 in the Sema3A receptor complex. (a) Association of L1 with the Sema3A receptor complex activates a chemorepulsive signal that is initiated via L1/NP-1 co-internalization. (b) In the absence of L1 (L1-deficient mice), the receptor complex is no longer functional and the repulsive signaling is blocked. (c) Simultaneous *cis* and *trans* interactions of L1 with NP-1 block endocytosis and modify the signaling pathway. Activation of the nNOS/cGMP pathway is involved in the switch from repulsive to attractive responses. (d) L1/NP-1 complex formation in the cell membrane is also necessary for triggering Sema3A attractive responses since wild-type L1Fc cannot switch Sema3A repulsion to attraction in the absence of neuronal L1 (L1-deficient mice) even though it binds NP-1.

### EXAMPLES

### Example 1: Material and Methods

### A. Co-culture experiments

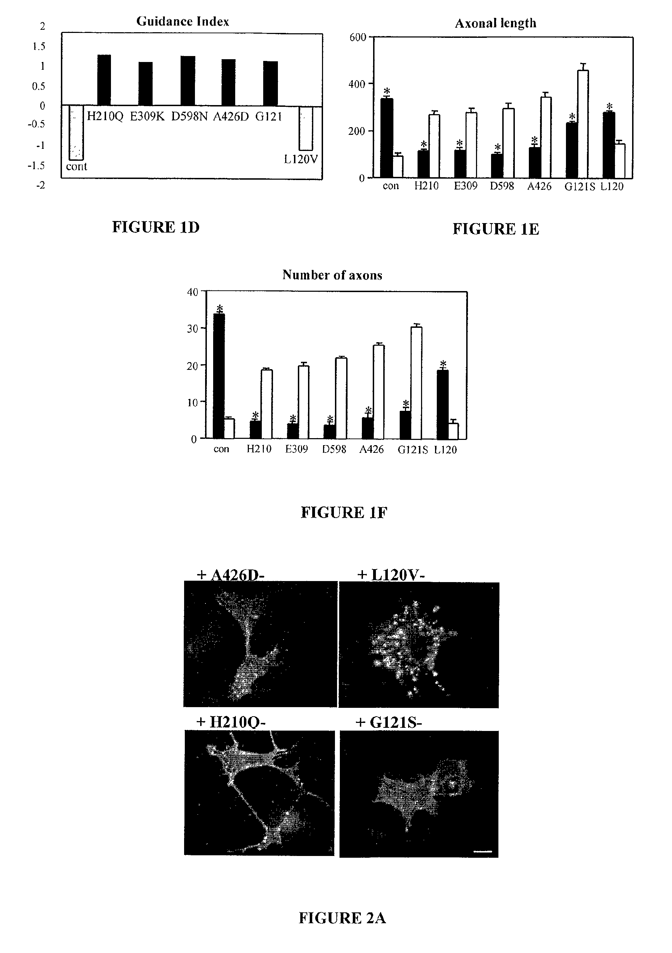

Blocks of cortex were dissected from P0 (postnatal day 0) to P2 mice and cut into 250 µm thick slices with a McIlwain tissue chopper. The ventral part of the cervical spinal cord was dissected and cut into 250 µm thick slices. Co-culture of cortical slices and ventral spinal cord explants were performed in a three-dimensional plasma clot, as described previously (Castellani et al., 2000). Cortical slices were oriented in the plasma clot as indicated in Figure 1B to give to axons equivalent chances to be deflected away or towards the spinal cord explant. Purified wild-type or mutant L1Fc chimeras were added to the culture medium (3 µg/ml). Inhibitors of the guanylyl cyclase (ODQ, 10⁻⁵ M, Sigma) and NOS (7NI, 10⁻⁴ M, Sigma) were added to the culture medium, either alone or with wild-type L1Fc (3 µg/ml). After one or two days in vitro (DIV), the cultures were fixed with 4 % paraformaldehyde (PFA) and analyzed by phase contrast microscopy (Axiovert 35-M, Zeiss). A qualitative guidance index was attributed to the co-cultures, to assess the degree of repulsive (negative values) or attractive (positive values) effects (Castellani et al., 2000). The index ranged from -2 (when most fibers grew away from the spinal cord explant) to +2 (when most fibers grew towards the explant), with intermediate situations (index of -1 and +1 for moderate guidance effects). An index of zero was attributed to the cultures showing no preferential growth. Quantitative analysis of axon length and number of axons growing from cortical slices was performed as described previously (Castellani et al., 2000). A proximal and a distal region, each of them covering a 600 µm-piece of cortical tissue were determined for each culture. All fibers extending from these two regions were counted and the length of the three longest fibers was measured, using computer analysis software (Visiolab 2000, Biocom). Immunofluorescence labeling of axons was performed using the mouse anti-phosphorylated neurofilament antibody SMI31 (Sternberger and Meyer Inc.), and Texas red-conjugated secondary anti-mouse antibody (see result obtained for number of axons and axonal length in the following tables 1 and 2).

**Table 1:**

| **Number of axons** | | |
|---|---|---|
| **Ctx/vSC** | **Away** | **Towards** |
| Control | 25.8 +/- 2 | 4.7 +/- 0.8,* |
| +L1FC | 5.8 +/- 0.7 | 22.6 +/- 1.6, * |
| +ODQ | 25.2 +/- 1.8 | 5.1 +/- 0.5, * |
| +L1Fc +ODQ | 24.2 +/- 1 | 6 +/- 0.4, * |
| +7NI | 25.6 +/- 1.6 | 5 +/- 0.6, * |
| +L1Fc +7NI | 26.1 +/- 1.1 | 5 +/- 0.4, * |

**Table 2:**

| **Axonal length** | | |
|---|---|---|
| **Ctx/vSC** | **Away** | **Towards** |
| Control | 411.2 +/- 5 | 213.4 + /-4, * |
| +L1FC | 198.6 +/ -4 | 346.8 +/ -5, * |
| +ODQ | 384.1 +/- 7 | 206.9 +/- 8, * |
| +L1Fc +ODQ | 410.5 +/- 3 | 207.1 +/- 7, * |
| +7NI | 407.6 +/- 5 | 203.1 +/- 6, * |
| +L1Fc +7NI | 408.2 +/- 4 | 213.2 +/- 4, * |

### B. Binding assays

The mutant L1Fc chimeras were produced and purified as described previously (De Angelis et al., 1999). Briefly, COS-7 cells were transiently transfected with 10 µg DNA per 150 mm culture dish, the soluble Fc chimeric protein was allowed to accumulate in the media for 6 days and was recovered and purified by protein A-Sepharose affinity chromatography. In the binding experiments, L1Fcs were crosslinked to anti-human Fc antibodies (50 µg/ml, Jackson Immunoresearch) for 1 hr at 37°C. Binding detection to NP-1 requested concentrations of L1Fcs 10 folds higher than L1. Antibody conjugated L1-Fc protein was then incubated with monolayers of COS7 cells transiently transfected with expression vectors encoding full length human L1 or myc-tagged NP-1 where the cytoplasmic domain is deleted (gift of J. Raper, 2000 and Renzi et al., 1999). Following incubation, the cells were fixed with 4 % PFA, and processed for double-immunofluorescence detection. NP-1 was detected using a mouse anti-c myc antibody (clone 9E10, Sigma) and L1 using a rat monoclonal anti-L1 antibody. Secondary antibody anti-c-myc (Texas-red conjugated anti-mouse antibodies) was used to detect NP-1, anti-L1 (Texas red conjugated anti-rat antibodies) to detect L1, and anti-Fc (FITC conjugated anti-rabbit antibodies) to detect bound L1Fc chimeras. After secondary antibody incubation, the cells were washed with PBS and mounted in Mowiol (Calbiochem).

### C. Two color aggregation assay

A two-color aggregation assay was developed, modified from the homophilic binding assay described by De Angelis et al. (1999). For the two-color assay, red and green fluorescent microsphere beads (0.6um, Duke Scientific Corps.) were coated with anti-human IgG antibody (Fc specific; Sigma, I-2136). 2.5 µg of wild type (red beads) or mutant (green beads) L1Fc-proteins were conjugated to 10 µl of antibody-coated beads by incubating for 2 hrs at 37°C. Excess unbound protein was removed by washing with PBS/5 % FCS. For each mutant, a 1:1 mixture of wild-type L1 and mutant L1 coated beads was prepared, dissaggregated to produce a single bead suspension and allowed to form mixed aggregates at 37°C with samples removed in duplicate over a 30-minute time course. Samples were diluted 1:5000 into ice cold PBS and the proportion of 2 colour aggregates was assessed using a fluorescence activated cell sorter (Becton Dickenson FACSort). Ten thousand particles were counted per sample and categorised as red only (WT-L1: WT-L1), green only (Mutant L1: Mutant L1) or mixed aggregates. Every mutant protein was assayed at least 3 separate times and standardised to a wild-type control conducted in parallel.

### D. Coimmunoprecipitation experiments

COS cells were transfected with various expression vectors coding for full-length HA-tagged NP-1, myc-tagged cyt-deleted NP-1, wild-type L1, and L1 mutant constructs (G121S, A426D, L120V) using a lipofection transfection method (lipofectamine). After 2 DIV, cells were lysed with immunoprecipitation buffer (IP buffer: Hepes 25mM, EDTA 5mM, MgCl2 1mM, PMSF 2mM, glycerol 10 % and triton X100 1 %, pH 7). The lysate was incubated at 4°C for 30 min, centrifuged for 5 min and pre-cleared with protein A-Sepharose for 2 hrs at 4°C. Samples were immunoprecipitated with mouse anti-HA (3 µl, clone 12CA5, Roche), rabbit anti-L1 antibodies (3 µl), mouse anti-myc antibodies (3 µl, 9E10, Sigma) coupled to rabbit anti-mouse antibodies on protein A preformed complexes for 16 hrs at 4°C. The beads were washed, and the precipitates analyzed by immunoblot using anti-HA (1:1000) and anti-L1 (1:1000) antibodies.

### E. Rhodamine-Dextran uptake

Neonatal cortical cells or COS7 cells transfected with expression vectors for L1 and NP-1 were incubated for 15 min with Rhodamine-Dextran (2 mg/ml) mixed to a solution of Sema3A-AP supernatant, Sema3A and peptide (10⁻⁵M), peptide alone (10⁻⁵M) or control supernatant. Cells were fixed with PFA 4 % and processed for immunocytochemistry as described in the above sections. To detect intracellular proteins, cells were permeabilized with methanol for 2 min.

### Example 2: Mutation L120V in Ig domain 1 of L1Fc abrogates the switch of Sema3A repulsion to attraction

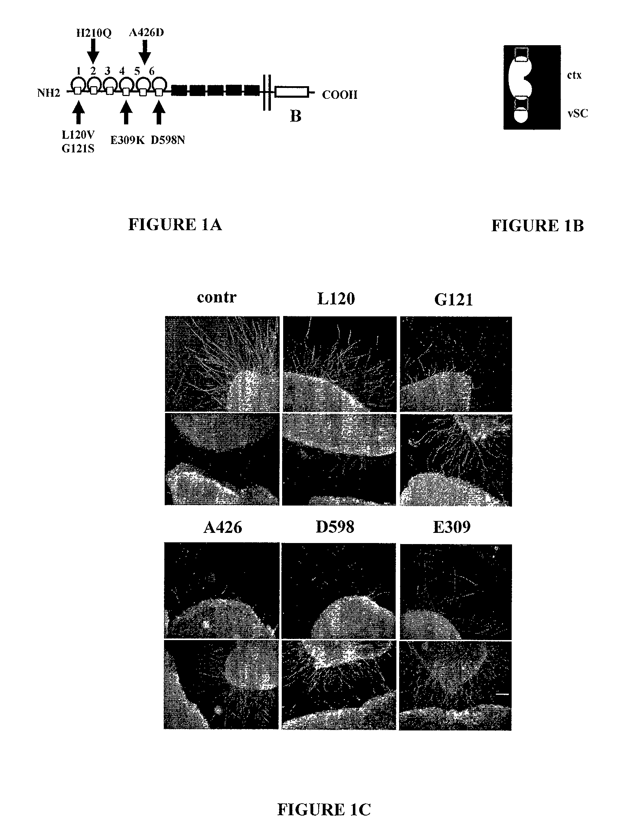

It has been previously developed an assay system using explants of ventral spinal cord co-cultured with cortical axons in order to determine the factors involved in axonal repulsion and attraction (Castellani et al., 2000). Using this system we have shown that Sema3A secreted from the ventral spinal cord repels cortical axons. In the presence of soluble wild-type L1Fc chimeric protein however, repulsion is switched to attraction (Castellani et al., 2000). In the present study, we aimed to identify the L1 interactions underlying the switching process. As Ig domains have been implicated in binding of Ig CAMs to many of their ligands (Brümmendorf and Rathjen, 1996) we hypothesized that they would also be required for L1Fc-mediated switching. Six L1Fc chimeras containing pathological missense mutations affecting specific Ig domains (De Angelis et al., 1999 and Figures 1A, 1B) were assessed for their ability to reverse Sema3A repulsion to attraction.

For each mutant, soluble Fc-chimeric protein was produced in mammalian cells and purified by protein A-Sepharose affinity chromatography. The purified L1Fc chimeras were subjected to SDS PAGE and immunoblotted with anti-Fc antibodies to verify their integrity. In the co-culture assay, five out of six L1-Fc proteins containing a single point mutation in Ig domains 1, 2, 3, 5 or 6 (G121S, H210Q, E309K, A426D and D598N, respectively) were found to efficiently induce switching of Sema3A repulsion to attraction (Figure 1C). As illustrated by both the qualitative guidance index and quantitative analysis, the majority of axons exhibited preferential growth towards the Sema3A source in the presence of the L1Fc chimera, rather than extending away from it as seen in the control (Figures 1D-1F, total of 147 co-cultures in three separate experiments). In contrast, one missense mutation, L120V in Ig domain 1, abrogated the reversal of Sema3A repulsion to attraction. When the L120V chimeric Fc protein was added to the culture medium, axons continued to be repelled from the spinal cord explant. This resulted in a negative guidance index of -1.3 attributed to the co-cultures, and a statistically significant preferential growth of fibers on the side opposing rather than facing the spinal cord explant (Figures 1C-1F). Thus, at least part of Ig domain 1 is required for L1Fc to switch the axonal response.

It has been aimed to identify the receptor on the cortical axons that binds L1Fc and initiates the reversal of Sema3A repulsion to attraction. L1 and/or NP-1 could fulfill this function, as they are both components of the Sema3A receptor complex and both interact in *trans* with L1 (Castellani et al, 2000). Plexin-A1 may also mediate the reversion of Sema3A as it associates with NP-1 in the Sema3A receptor. Other known L1 ligands, including Ig CAMs F3/contactin and TAG-1/axonin are also candidate receptors for L1Fc. Previous work has indicated that the mutations described above can selectively affect either homophilic binding or heterophilic binding to F3 or axonin-1 (De Angelis et al, 1999). Two of these mutations G121S and A426D reduce homophilic binding and heterophilic binding to F3, axonin-1 and the human homologue of axonin-1, TAX-1. H210Q and E309K affect homophilic or heterophilic binding respectively. D598N has a very modest effect on homophilic binding but significantly reduces heterophilic binding to F3 and axonin-1, though not to TAX-1. L120V does not affect homophilic or heterophilic binding. Therefore we were able to compare the ability of the mutated chimeras to reverse the Sema3A repulsion with their binding capabilities.

### Example 3: IgCAMs including L1 are not axonal receptors for L1Fc in the reversion of Sema3A repulsion

Four mutations (E309K, D598N, A426D and G121S) that were found to alter L1Fc binding to the Ig CAMs F3 and axonin-1switched Sema3A from repulsion to attraction to the same extent as wild type L1Fc. Thus, Ig CAMs F3 and TAG-1 (the rodent homologue of axonin-1) are unlikely to be axonal receptors for L1Fc in the reversion process. Mutations A426D, H210Q and G121S were previously found to reduce homophilic binding when individual mutant L1Fc proteins were bound to themselves (De Angelis et al., 1999). In the present study however, mutated L1Fc proteins were required to interact with wild-type L1 expressed on growth cones. To formally exclude a role for homophilic binding of L1 in the initiation of the reversion process, it was therefore necessary to examine whether homophilic binding of the mutated L1Fc chimeras to wild-type L1 was also disrupted. Mutated L1Fcs were therefore incubated with COS7 cells transfected with full-length wild-type L1 cDNA and binding was assessed by immunofluorescence. In parallel, homophilic binding of mutant/mutant and mutant/wild-type L1Fcs was compared in a two-color FACS aggregation assay, allowing a quantitative assessment to be made (see Methods). Interestingly, the immunofluorescence studies showed that the A426D and H210Q L1Fc chimeras bound to wild-type L1 (Figure 2A). Similarly, in the two color FACS assay homophilic binding was restored to 74.4% and 90.5 % of wild-type levels for A426D and H210Q respectively (Figure 2B). In contrast, the mutation G121S, almost abolished homophilic binding to wild-type L1 in both assays (Figures 2A, 2B). This mutation however still promoted the L1Fc induced switching of Sema3A repulsion to attraction in the co-culture assay. Thus, the ability of L1Fc to switch Sema3A repulsion is not dependent on homophilic binding. Consequently, the *trans* interaction of L1Fc with L1 in the receptor complex is not required for the change in growth cone responsiveness to Sema3A. In support of this conclusion we also observed that the L120V mutated L1Fc, retained 100 % homophilic binding capability but failed to reverse Sema3A effect (Figures 2A, 2B).

### Example 4: NP-1, but not Plexin-A1, is the axonal receptor for L1Fc in the switch of Sema3A repulsion

To determine whether soluble L1Fc binds plexin-A1, COS7 cells were transiently transfected with a vector encoding Plexin-A1 fused to a VSV-tag (Rohm et al., Mech Dev., 93:95-104, 2000) and incubated with wild-type L1Fc protein. As a positive control, chimeric protein was also incubated with COS7 cells transiently transfected with full-length wild-type L1. Immunodetection of L1Fc with anti-Fc antibodies showed clear binding of the chimera on L1 expressing cells but no binding to Plexin-A1 expressing cells (Figure 3). Therefore it is unlikely that Plexin-A1 is the axonal binding partner of L1Fc in the switch of Sema3A repulsion to attraction.

Next, we examined the ability of the mutant L1Fc chimeras to bind NP-1. COS7 cells were transfected with a vector encoding the extracellular and transmembrane domains of NP-1 fused to a myc tag (ΔNP-1, Renzi et al., J. Neurosci., 15:7870-7880, 1999), and incubated with the mutant L1Fc chimeras. Binding was detected by immunofluorescence. The assays showed that mutant chimeras containing G121S, H210Q, E309K, D598N and A426D bound NP-1 expressing cells. In contrast, L120V-L1Fc did not bind NP-1. Binding of L120V, G121S and A426D L1Fcs to NP-1 is illustrated in Figure 3. As L120V-L1Fc does not interact with NP-1 in binding assays nor switches Sema3A repulsion to attraction in the co-culture experiments, this strongly suggests that NP-1 is the axonal receptor for L1Fc-mediated switching (See Model in Figure 9A).

### Example 5: A six amino-acid peptide mimics the L1Fc reversing bioactivity

It has been postulated that only a restricted sequence within Ig1 domain of L1 is involved in the interaction with NP-1. G121S, a mutation that is likely to disrupt Ig domain 1 conformation (Bateman et al, EMBO J., 15:6050-6059, 1996), modified neither NP-1 binding nor the switching process, whereas the neighboring mutation L120V, affecting a surface residue, abrogated both. According to this hypothesis, isolated peptides should mimic L1Fc in reversing Sema3A repulsion to attraction. Since the G121S mutation did not abrogate L1Fc switching, this amino-acid should be dispensable for peptidic bio-activity. Based on these criteria, a six amino-acid peptide, FASNKL₁₂₀ (SEQ ID N° 2), with free NH2 and COOH terminals (Schafer-N, Copenhagen DK), was designed and tested in the co-culture assay (Figures 4A, 4B). It successfully converted Sema3A-induced chemorepulsion into attraction at 10⁻⁶M concentration (Figure 4B).

The inventors have also demonstrated that the five amino-acid peptide ASNKL₁₂₀ and the ten amino-acid peptide FASNKLGTAM (SEQ ID N° 3) successfully converted Sema3A-induced chemorepulsion into attraction in the same conditions as tested for the six amino-acids peptide FASNKL₁₂₀ and so also mimic the L1Fc reversing bioactivity (data not shown).

Furthermore, mimicking the human mutation by replacing the C-terminal leucine¹²⁰ by valine in the peptide induced a 100-fold decrease in its converting activity. A residual effect was only observed at 10⁻⁴M (Figure 4B). Thus, the six amino-acid peptide FASNKL₁₂₀ and the five amino-acid peptide ASNKL₁₂₀contains the L1 sequence that binds to NP-1 and leucine¹²⁰ is a critical residue for the interaction.

### Example 6: The pathological mutation L120V disrupts the L1/NP-1 receptor complex for Sema3A

It has been showed previously that on the cell membrane, L1 and NP-1 associate in a common molecular complex that serves as a receptor for Sema3A (Castellani et al., 2000). We investigated the effects of L1 mutations on this complex formation. First, we determined whether L1 and NP-1 extra-cellular domains are required for the *cis*-interaction. COS7 cells were co-transfected with vectors encoding NP-1 lacking its cytoplasmic domain (ΔNP-1, Renzi et al., 1999) and full-length wild-type L1. The cells were lysed and proteins immunoprecipitated by protein A-Sepharose coupled to rabbit anti-mouse and mouse anti-myc antibodies. Immunoprecipitated proteins were identified by western blot analysis using anti-L1 and anti-NP-1 antibodies. Co-immunoprecipitation of both ΔNP-1 and L1 indicated that *cis* association of the extracellular domains is required for the formation of the L1/NP-1 complex (Figure 5B, lane 3). Second, we expressed the mutated L1 proteins in COS7 cells and verified their cell surface expression by immunofluorescence (Figure 5A). Third, COS7 cells were co-transfected with vectors encoding full-length HA-tagged NP-1 and wild-type or mutant L1. Cells were lysed and immunoprecipitated with protein A-Sepharose coated with anti-HA antibodies. Recovered proteins were subjected to SDS-PAGE, transferred and immunoblotted with anti-L1 and anti-HA antibodies. The results show that L1 containing missense mutations A426D and G121S successfully immunoprecipitated NP-1 whereas L1 containing L120V did not (Figure 5 lanes 4-6). Therefore both *trans* and *cis* interactions of L1 with NP-1 depend on the same binding site within the six amino-acid sequence of Ig1 domain described above.

A possible molecular mechanism by which soluble L1 and its mimetic peptide could exert their modulatory effect on Sema3A signaling is that they compete with transmembrane L1 in the L1/NP-1 complex. To address this issue we examined in immunoprecipitation experiments the L1/NP-1 complex in conditions mimicking either a repulsive (Sema3A alone) or an attractive (Sema3A combined to the peptide) environment. We observed that the presence of the peptide did not abrogate the *cis* interaction between L1 and NP-1 (Figure 5C), as L1 was found to co-precipitate with NP-1 in the presence of Sema3A alone or combined to the peptide, as well as in the control conditions. Since we could not evidence competition between *cis* and *trans* interactions of L1 with NP-1, we attempted to identify other mechanisms that might account for switching axonal repulsion to attraction.

### Example 7: cis and trans L1/NP-1 interactions control Sema3A-induced endocytosis

Endocytosis and re-organization of the Sema3A receptor complex within axon terminals were shown to accompany Sema3A-induced growth cone collapse (Fournier et al., 2000). It has been found that soluble L1 prevents growth cone collapse in response to Sema3A (Castellani et al., 2000). Therefore it has been tested whether L1 mimetic peptide was able to block endocytosis. To visualize endocytosis we examined the uptake of Rhodamine-Dextran applied on live cortical cultures in various conditions. Cells were treated for 15 min with Rhodamine-Dextran together with Sema3A alone, Sema3A combined with the L1 mimetic peptide, peptide alone or untreated as control conditions. Strikingly, uptake of Rhodamine-Dextran was exclusively observed when cells were treated with Sema3A alone. Under this condition, spots of internalized fluorescence were observed along axon shafts and surrounding the cell bodies. In contrast, no uptake of Rhodamine-Dextran could be detected following all other treatments (Figure 6A). This therefore indicated that the peptide mimicking soluble L1 blocks Sema3A-induced endocytosis. To correlate this process with L1/NP-1 *cis* and *trans* interactions, the experiments of Rhodamine-Dextran uptake were performed on COS7 cells transfected to co-express L1 and NP-1 (Figure 6B). As in cortical cells Sema3A alone but not Sema3A combined with the mimetic peptide induced internalization of the Rhodamine-Dextran in co-transfected cells. No fluorescent labeling was detected following treatment with the peptide alone or in the control conditions (Figure 6B).

In parallel, cells submitted to the same treatments were processed for immunocytochemical detection of L1 and NP-1. Interestingly, we observed that the cell-surface expression of both L1 and NP-1 was abolished by the Sema3A treatment (Figure 7A). In contrast, the two proteins were detected in all other conditions, including treatment by Sema3A combined to the peptide (Figures 7B-7E). Furthermore, in the Sema3A-treated cultures L1 and NP-1 immunoreactivities were detected after permeabilization, as co-localized cytoplasmic patches (Figures 7F-7H). These results indicated first that Sema3A induced a co-internalization of L1 and NP-1, and second that this process was blocked by the switching peptide.

This finding strongly suggested that L1 function could be to ensure the appropriate endocytosis of NP1 associated to Sema3A-induced axonal repulsion In support, we verified that Sema3A is unable to induce endocytosis and internalization of NP-1 in the absence of L1. COS7 cells were transfected to express NP-1 alone and the uptake of Rhodamine-Dextran was examined following Sema3A treatment. As expected, NP-1 endocytosis did not occur in the absence of L1 since immunochemical labeling confirmed that NP-1 remains on the surface of cells treated with Sema3A and that Sema3A - induced endocytosis did not occur in the absence of L1 (data non shown).

### Example 8: L1/NP-1 trans interaction activates NO synthesis and subsequently increases cGMP levels

Previous studies have shown that Sema3A chemorepulsion is converted into attraction by an increase in the internal level of cGMP (Song et al., 1998). In order to explore the possibility that L1/NP-1 *trans* interaction activates the cGMP pathway to specify a Sema3A attractive effect, we used a pharmacological approach. 1H-(1,2,4)oxadiazolo(4,3-a)quinoxalin-1-one (ODQ) prevents cGMP synthesis by specifically blocking soluble guanylyl cyclase. ODQ was added to co-cultures of spinal cord and cortical explants either alone or in combination with L1Fc (Figure 8). Addition of ODQ alone (10⁻⁵M) did not modify chemorepulsion compared with control (guidance index of -1.6 and -1.7, respectively). The number and the length of axons differed significantly between the growth towards and away from the spinal cord explant (Figure 8). L1Fc alone switched repulsion to attraction as expected (guidance index of +1.5). However, when ODQ was combined to L1Fc (3µg/ml), reversion was prevented (guidance index of -1.5). This suggests that cGMP synthesis is necessary for L1Fc to reverse Sema3A repulsion. Nitric oxide (NO) is a well-known activator of guanylyl cyclase (Hanafy et al., Med. Sci. Monit., 7:801-819, 2000). We examined whether activation of the neuronal NO synthase (nNOS) is also required for the reversion process. A specific inhibitor of NOS, 7 nitroindazole (7NI) was added to cortical slices and ventral spinal cord co-cultures. As observed with ODQ, 7NI alone (10⁻⁴ M) did not modify the chemorepulsion observed in the controls (guidance index of -1.6 and -1.7, respectively, Figure 8), but it did block L1Fc-mediated chemoattraction. Most axons grew preferentially away from the spinal cord explants, as in controls (Figure 8). This suggests that L1-NP-1 *trans* interaction triggers NO synthesis and a subsequent activation of cGMP guanylyl cyclase to induce the reversion of Sema3A repulsion to attraction.

### Example 9: Peptide mimicking soluble L1 blocks Sema3A-induced endocytosis

Endocytosis and re-organization of the Sema3A receptor complex within axon terminals were shown to accompany Sema3A-induced growth cone collapse (Fournier et al., 2000).

It has been found that soluble L1 prevents growth cone collapse in response to Sema3A (Castellani et al., 2000).

### Experiments:

Therefore, it has been tested whether L1 mimetic peptide was able to block endocytosis. To visualize endocytosis the uptake of Rhodamine-Dextran applied on live cortical cultures has been examined in various conditions. Cells were treated for 15 min with Rhodamine-Dextran together with Sema3A alone, Sema3A combined with the L1 mimetic peptide, peptide alone or untreated as control conditions. Strikingly, uptake of Rhodamine-Dextran was exclusively observed when cells were treated with Sema3A alone. Under this condition, spots of internalized fluorescence were observed along axon shafts and surrounding the cell bodies. In contrast, no uptake of Rhodamine-Dextran could be detected following all other treatments.
Conclusion: the peptide mimicking soluble L1 blocks Sema3A-induced endocytosis.

To correlate this process with L1/NP-1 interactions, the experiments of Rhodamine-Dextran uptake were performed on COS7 cells transfected to co-express L1 and NP-1. As in cortical cells, Sema3A alone but not Sema3A combined with the mimetic peptide induced internalization of the Rhodamine-Dextran in co-transfected cells. No fluorescent labeling was detected following treatment with the peptide alone or in the control conditions.

In parallel, cells submitted to the same treatments were processed for immunocytochemical detection of L1 and NP-1. We observed that the cell-surface expression of both L1 and NP-1 was abolished by the Sema3A treatment (Figure 7A). In contrast, the two proteins were detected in all other conditions, including treatment by Sema3A combined to the peptide. Furthermore, in the Sema3A-treated cultures, L1 and NP-1 immunoreactivities were detected after permeabilization, as co-localized cytoplasmic patches.

Thus, Sema3A induces a co-internalization of L1 and NP-1. The endocytsis of the two proteins is blocked by the peptide.

On the basis of these results, two cellular tests for the screen of active peptides or molecules can be performed.
- The test N° 1 consists in cultering cells expressing L1 and NP-1 (cell lines transfected with expression vectors or neuronal cells) in the presence of Sema3A and rhodamine-dextran (or FITC-dextran or dextran coupled to fluorescent molecules). The test is used to screen for molecules that prevent the internalization of the dextran induced by the Sema3A treatment.
- In the test N° 2 the cells expressing L1 and NP-1 (cell lines or neuronal cells) are treated with Sema3A and screened with candidate molecules. Immunocytochemistry for L1 and/or NP-1 is performed to detect cell surface expression of the protein(s). No labeling should be detected following the Sema3A treatment (control conditions). The test is used to screen for molecules that maintain a cell surface expression.

These results provide evidence that Sema3A repulsion is associated with L1-mediated NP-1 endocytosis. Switching of Sema3A repulsion into attraction is initiated via a specific *trans* interaction of L1 with NP-1 that blocks endocytosis. This is followed by downstream activation of the intracellular NO/cGMP pathway. One individual missense mutation L120V located in Ig domain 1 of L1, completely prevented the switch from repulsion to attraction and disrupted both the *trans* and *cis* L1/NP-1 interactions. In contrast, other mutant L1 proteins that had previously been shown to alter homophilic and heterophilic binding to other IgCAMs still effectively reversed Sema3A repulsion and bound NP-1 (Figure 9A). A five and a 6 amino-acid peptide derived from Ig1 domain was sufficient to convert Sema3A-induced repulsion, suggesting that the L1/NP-1 binding site is restricted to FASNKL amino-acid sequence. In the light of these findings we propose a model whereby *cis* and *trans* L1/NP-1 interaction may control the responsiveness of growth cones to Sema3A.

### The L1/NP-1 binding site is located in L1 Ig1 domain

The Sema3A receptor complex has been shown to contain a binding subunit (NP-1) and a signal transducer (Plexin) (Tamagnone et al., 2000). Recently, we proposed that L1 is also a component of this receptor complex (Castellani et al., 2000). Previous studies have shown that several Ig domains of L1 are required for homophilic and heterophilic Ig CAM binding (Appel et al., Neurosci., 13:4764-4775, 1993 ; Su et al., Nat Genet., 7:408-413, 1998 ; Haspel et al., J Neurobiol., 15:287-302, 2000). This is highlighted by the number of pathological mutations in Ig domains that affect these interactions (De Angelis et al., 1999). In contrast, our finding that several of these mutations did not alter the L1/NP-1 interaction suggested that a restricted region of L1 might define the binding site for NP-1. This idea was confirmed by our results showing that a 6 amino-acid peptide could mimic the integral L1Fc in reversing Sema3A repulsion to attraction. As demonstrated by the experiments using L1Fc with the mutation L120V and the peptide, modifying residue leucine¹²⁰ to valine completely disrupted both the L1-NP-1 interaction and the reversion process. Together, these findings indicate that the binding site for NP-1 is contained in the FASNKL sequence, with the leucine being a crucial residue.

Our previous finding suggested that L1Fc may act independently of the Sema3A signaling pathway, activating through homophilic binding, an intracellular cascade that results in increased cGMP levels and a subsequent switch from repulsion to attraction (Castellani et al., 2000; He, Neuron, 27:191-193, 2000). Although we found that L1Fc does indeed activate guanylyl cyclase, this is not dependent on homophilic interaction as shown by the experiments using G121S-L1Fc. We therefore propose that the switch occurs through a selective interaction with NP-1 in the receptor complex. Interestingly, *cis* and *trans* L1-NP-1 interactions were both found to use the same binding site. However, soluble and transmembrane L1 do not compete for the binding to NP-1. This is consistent with our previous finding that indicated that L1Fc did not enable L1-deficient axons to become susceptible to Sema3A-induced chemorepulsion. In addition, L1Fc was also unable to induce attraction of L1-deficient axons (Castellani et al., 2000). L1-NP-1 *cis* interaction is therefore a pre-requisite for the Sema3A receptor complex to transduce both repulsive and attractive signals. Thus, dimeric forms of L1 and NP-1 in the membrane might allow both *trans* and *cis* interaction to occur simultaneously.

### Dual function of L1/NP-1 cis and trans interactions on endocytosis and control of Sema3A-induced repulsive and attractive behaviors

The present work demonstrates that transmembrane L1 mediates NP-1 endocytosis associated to Sema3A-induced repulsive responses (Fournier et al., 2000). Conversely, *trans* interaction of soluble L1 mimetic peptide blocks L1/NP-1 receptor complex endocytosis, switching Sema3A from repulsion to attraction. One can correlate endocytosis with the retraction of filopodial and lamellipodial structures that occurs during repulsive contacts. How blockade of endocytosis leads to the opposite effect remains presently unclear. One possibility is that persistence of the ligand/receptor interaction subsequently stabilizes the growth cone structures. Whatever the mechanisms at play they must ultimately result in intracellular signaling modifications. Our results are consistent with recent findings indicating that local protein synthesis and degradation are necessary for guidance cues to steer growth cone responses (Campbell and Holt, Neuron, 32:1013-1026, 2001). As evidenced in several recent works, receptor endocytosis controls levels of degradation by the proteasome, cell surface expression and intracellular downstream signaling (Lloyd et al., Cell., 25:261-269, 2002 ; Soubeyran et al., Nature, 14:183-187, 2002). Thus, control of endocytosis is likely to be instructive in the equilibrium between synthesis and proteolysis in the growth cone and determinant in the nature of the responses elicited by guidance cues.

### The switch of Sema3A chemorepulsion to attraction requires an activation of the NO/cGMP pathway

Blocking soluble guanylyl cyclase in co-culture assays using a pharmacological inhibitor prevented L1Fc from reversing Sema3A repulsion to attraction. The requirement of cGMP is consistent with previous work (Song et al., 1998) that established increased levels of cGMP as pivotal to Sema3A signaling. In the developing cortex, Sema3A has been shown to exert a dual effect, repelling axons whilst attracting dendrites (Polleux et al., Nature, 6:567-573, 2000). Based on its asymmetric subcellular localization in the cell body, it was proposed that guanylyl cyclase was responsible for the specific attractive behaviour of the dendritic growth cones to Sema3A (Polleux et al., 2000). Our findings however indicate that cortical axons also express the appropriate molecular machinery to display attractive responses to Sema3A. This may be due to the different stages in development that the cortical neurons were examined in each study (postnatal neurons were used in the present work compared with embryonic cells in the Polleux study). Alternatively, changes in either the localization of the guanylyl cyclase during maturation or up or downstream events may account for the differences observed in the response of cortical axons and dendrites.

Furthermore, our findings also suggest that NO acts as a key messenger in the reversal of Sema3A chemorepulsion to attraction. Switching of Sema3A-induced responses may therefore depend closely upon the maturation of nNOS expression. In the mouse cerebral cortex, nNOS immunoreactivity is present from late embryonic stages, peaking during the first postnatal week, and then declining to adult levels (Oermann et al., Anat. Embryol. (Berl) 200:27-41, 1999). NO is thought to regulate the refinement of immature synaptic connections (Wu et al., Science, 265:1593-1596, 1994) and some forms of adult synaptic plasticity (reviewed by Kiss and Vizi, Trends Neurosci., 24:211-215, 2001). Our observations suggest a novel function of NO in patterning of neuronal projections by modulating axonal responses to guidance cues, particularly in postnatally developing cortical tracts.

### Modulation of Sema3A signaling and axonal pathfinding

It is likely that modification of Sema3A-mediated responses by soluble L1 also occurs in vivo. The dynamic changes within the Sema3A receptor complex may occur as a result of contact between the growth cone and either membrane-bound L1 or L1 released from the cell surface. In support, several studies reported proteolytic cleavage of L1 by plasmin(ogen) and by ADAM10, a member of the disintegrin metalloproteinase family (Nayeem et al., J. Cell. Sci., 112:4739-4749, 1999 ; Gutwein et al., J. Biol. Chem., 19:15490-15497, 2000 ; Mechtersheimer et al., J. Cell. Biol., 12:661-674, 2001). Furthermore, membrane-proximal cleavage of L1 is detected in the postnatal brain and might be required for L1-dependent migratory processes (Mechtersheimer et al., 2001). With respect to axon guidance, our data allow us to propose that soluble L1 may act in at least two non-exclusive ways. On the one hand soluble L1 can induce the conversion of Sema3A-induced responses from repulsion to attraction in neighboring growth cones. On the other hand, shedding of the L1 ectodomain may disrupt the Sema3A receptor complex, thereby desensitizing the growth cone to Sema3A. This is consistent with recent findings that revealed metalloprotease activity as the determining factor in axon responsiveness to other types of guidance cues (Galko et al., Science, 25:1365-1367, 2000 ; Hattori et al., Science, 25:1360-1365, 2000). Switched responses to a guidance cue of the Slit family have recently been shown to be operative in directing the migration of muscle pioneers (Kramer et al., Science, 292:737-740, 2001). It is therefore tempting to speculate that switching Sema3A responsiveness is important during the formation of neuronal projections.

### Sema3A signaling and the human disease associated with L1 mutations

Interestingly, our data may elucidate molecular events that could contribute to the human disease pathology. Several theories have been proposed to explain the pathological defects associated with the L120V mutation. The first proposal is that this mutation generates a nucleotide sequence with a potential donor splicing site (De Angelis et al., 1999). Secondly, it has been suggested that the L120V mutation affects extracellular matrix interactions as it was able to disrupt L1 binding to the chondroitin sulfate proteoglycan Neurocan in in vitro assays (Oleszewski et al., J. Biol. Chem., 3:3478-3485, 2000). Our data would favor a third possibility whereby some aspects of the human pathology may be the result of a defective L1/NP-1 interaction affecting Sema3A signal transduction, thus leading to axon guidance defects during the development of cortical fiber tracts. Since we found that L1 mediates endocytosis of the Sema3A receptor complex, any type of mutations that affects L1 cell surface expression and trafficking can potentially alter the Sema3A signal transduction. This therefore increases the spectrum of mutations susceptible to disturb axonal responses to Sema3A during the development of neuronal projections.

## Claims

1. A peptide comprising SEQ ID N° 1, preferably, inducing attraction of the axonal growth, in particular in presence of the semaphorin (Sema3A, L1 and NP-1 proteins.

2. The peptide of claim 1 comprising no more than 10 amino acids.

3. The peptide of claim 1 consisting of SEQ ID N° 1.

4. The peptide of claim 1, comprising or consisting of SEQ ID N° 2.

5. The peptide of claim 1, comprising or consisting of SEQ ID N° 3.

6. An inducer of axonal growth comprising the peptide of anyone of claims 1 to 5.

7. A method for identifying a compound able to bind the NP-1 protein, comprising the steps of:
a) contacting said compound with a sample comprising the NP-1 protein, before, after or concomitantly with contacting said sample with the L1-Fc protein or the peptide of any of claims 1 to 5,
b) studying the binding between NP-1 and L1-Fc or said peptide, the binding of said compound to the NP-1 protein being deduced from the alteration of the binding of NP-1 with L1-Fc or said peptide observed in absence of said compound.

8. The method of claim 7, wherein said sample also comprises the L1 protein.

9. The method of claim 7 or 8, wherein said NP-1 protein is a recombinant protein.

10. The method of anyone of claims 7 to 9, wherein said sample comprises a cell expressing the NP-1 protein at its surface.

11. The method of any of claims 7 to 10, wherein said sample comprises a cell expressing the NP-1 and L1 proteins at its surface.

12. The method of claim any of claims 7 to 11, wherein said L1-Fc protein or said peptide is labeled and said binding of NP-1 protein with said L1-Fc protein or said peptide is assayed by level of signal associated with said NP-1 protein.

13. The method of any of claims 7 to 12, wherein said alteration of binding of NP-1 with L1-Fc or said peptide is assayed by measuring a secondary signal appearing in the presence of said binding.

14. The method of claim 13, wherein said secondary signal is induced in the presence of Sema3A.

15. The method of claim 14, wherein said secondary signal is the reversion of the repulsory effect of Sema3A on axonal growth.

16. The method of claim 14, wherein said secondary signal is the activation of NO synthesis.

17. The method of claim 14, wherein said secondary signal is an increased production of cGMP.

18. A method for attracting /modulating the direction of axonal growth by a cell suited to promote axonal growth, submitted to a Sema3A flow, comprising the step of contacting said cell to the peptide of any of claims 1 to 5, or the inducer of claim 6.

19. A method for identifying a compound able to revert the repulsory effect of Sema3A on the axonal growth from a suited cell, comprising the steps of contacting said cell expressing L1 and NP-1 at its surface with said compound in the presence of Sema3A, and studying the increase in NO synthesis in said cell.

20. A method to screen for molecules that prevent the internalization of the dextran induced by the Sema3A treatment or to screen for molecules capable of blocking Sema3A- induced endocytosis by cells, **characterized in that** said method comprises the following steps of:
a) culturing cells expressing L1 and NP-1 in the presence of Sema3A, a labelled dextran and the molecule to be assayed ; and
b) visualizing or determining if said labelled dextran has been internalized into the cells ;
c) selecting the assayed molecule if no uptake of the labelled dextran by the cells could be detected.

21. A method to screen for molecules capable of blocking Sema3A- induced co-internalization of L1 and NP-1 proteins, to screen molecules capable of maintaining cell surface expression of L1 and NP-1 proteins or to screen molecules capable of blocking Sema3A- induced endocytosis, **characterized in that** said method comprises the following steps of:
a) culturing cells expressing L1 and NP-1 in the presence of Sema3A and the molecule to be assayed ; and
b) visualizing or determining if said L1 and NP-1 proteins have been co-internalized into the cells using an immunocytochemical method in presence of antibodies directed against L1 and NP-1 proteins ;
c) selecting the assayed molecule if L1 and NP-1 proteins could be detected at the surface of the cells.

22. Use of the peptide of anyone of claims 1 to 5, or the inducer of claim 6, for the manufacture of a drug for neuronal/axonal regeneration.

23. Use of the peptide of anyone of claims 1 to 5, or the inducer of claim 6, for the manufacture of a drug for treating neurodegenerative diseases.

24. Use of the peptide of anyone of claims 1 to 5, or the inducer of claim 6, as a targeting agent, in the manufacture of a composition to be used in cell therapy.


SEQUENCE LISTING

<110> Centre National de la Recherche Scientifique - CNRS
Université d'Aix-Marseille 2

<120> New peptides capable of inducing attraction of the axonal growth,
particularly in presence of the semaphorin, and their use for
treating neurodegenerative diseases

<130> D19980

<160> 3

<170> PatentIn version 3.1

<210> 1
<211> 5
<212> PRT
<213> Artificial sequence

<220>
<223> Description of the Artificial sequence : Derived from the human
amino-acid sequence of L1 Ig1 domain

<400> 1

Ala Ser Asn Lys Leu

1               5


<210> 2
<211> 6
<212> PRT
<213> Artificial sequence

<220>
<223> Description of the Artificial sequence : Derived from the human
amino-acid sequence of L1 Ig1 domain

<400> 2

Phe Ala Ser Asn Lys Leu
1               5


<210> 3
<211> 10
<212> PRT
<213> Artificial sequence

<220>
<223> Description of the Artificial sequence : Derived from the human
amino-acid sequence of L1 Ig1 domain

<400> 3

Phe Ala Ser Asn Lys Leu Gly Thr Ala Met
1               5                   10